(19) Europäisches Patentamt
European Patent Office
Office européen des brevets

(11) **EP 4 026 566 B1**

(12) **EUROPEAN PATENT SPECIFICATION**

(45) Date of publication and mention
of the grant of the patent:
**31.07.2024 Bulletin 2024/31**

(21) Application number: **20861057.6**

(22) Date of filing: **13.05.2020**

(51) International Patent Classification (IPC):
*A61K 9/113* (2006.01)    *A61K 9/28* (2006.01)
*A61K 9/20* (2006.01)    *A61K 9/48* (2006.01)
*A61K 9/16* (2006.01)    *A61K 38/29* (2006.01)
*A61K 47/44* (2017.01)    *A61K 47/22* (2006.01)
*A61K 47/54* (2017.01)    *A61P 19/10* (2006.01)

(52) Cooperative Patent Classification (CPC):
**A61K 9/113; A61K 9/1617; A61K 9/2054;
A61K 9/2866; A61K 38/29; A61K 47/22;
A61K 47/44; A61K 47/541; A61K 47/554;
A61P 19/10**

(86) International application number:
**PCT/KR2020/006255**

(87) International publication number:
**WO 2021/045345 (11.03.2021 Gazette 2021/10)**

(54) **ORAL PHARMACEUTICAL COMPOSITION COMPRISING TERIPARATIDE AND METHOD FOR PREPARING SAME**

ORALE PHARMAZEUTISCHE ZUSAMMENSETZUNG MIT TERIPARATID UND VERFAHREN ZU IHRER HERSTELLUNG

COMPOSITION PHARMACEUTIQUE PAR VOIE ORALE COMPRENANT DU TÉRIPARATIDE ET SON PROCÉDÉ DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.09.2019 KR 20190110078**

(43) Date of publication of application:
**13.07.2022 Bulletin 2022/28**

(73) Proprietor: **Icure BNP Co., Ltd.
Chungcheongbuk-do 27643 (KR)**

(72) Inventors:
• **CHOI, Young Kweon
Seoul 06191 (KR)**
• **CHANG, Kwan Young
Seoul 07649 (KR)**
• **LEE, Jae Bum
Seoul 03709 (KR)**
• **KANG, Seohee
Seoul 03328 (KR)**

• **SEO, Yuseon
Hwaseong-si, Gyeonggi-do 18487 (KR)**
• **NA, Hyerim
Seoul 07393 (KR)**

(74) Representative: **Franke, Dirk
Franke & Partner
Patent- und Rechtsanwälte
Widenmayerstraße 25
80538 München (DE)**

(56) References cited:
CN-A- 102 125 520    KR-A- 20190 070 653
KR-B1- 101 796 604    KR-B1- 101 895 237
US-A1- 2006 052 305    US-A1- 2008 119 408

- PARK JIN WOO ET AL: "Ionic complex of risedronate with positively charged deoxycholic acid derivative: evaluation of physicochemical properties and enhancement of intestinal absorption in rats", ARCHIVES OF PHARMACAL RESEARCH, NATL. FISHERIES UNIVERSITY , PUSAN, KR, vol. 37, no. 12, 20 November 2013 (2013-11-20), pages 1560 - 1569, XP035377108, ISSN: 0253-6269, [retrieved on 20131120], DOI: 10.1007/S12272-013-0297-X
- GUO, Y. ET AL: "The applications of Vitamin ETPGS indrug delivery", EUROPEAN JOURNAL OF PHARMACEUTICAL SCIENCES, vol. 49, no. 2, 26 February 2013 (2013-02-26), pages 175 - 186, XP028531143
- HWANG, S. R.: "Preparation and in vivo evaluation of an orally available enteric-microencapsulated parathyroid hormone (1-34)-deoxycholic acid nanocomplex", INTERNATIONAL JOURNAL OF NANOMEDICINE, vol. 11, 31 August 2016 (2016-08-31), pages 4 231 - 4246, XP055799519
- PANGENI, R. ET AL: "Multiple nanoemulsion system for an oral combinational delivery of oxaliplatin and 5-fluorouracil: preparation and in vivo evaluation", INTERNATIONAL JOURNAL OF NANOMEDICINE, vol. 11, 30 November 2016 (2016-11-30), pages 6379 - 6399, XP055558169
- QIAO, H.: "Fabrication and in vitro/in vivo evaluation of amorphous andrographolide nanosuspensions stabilized by d-alpha-tocopheryl polyethylene glycol 1000 succinate/sodium lauryl sulfate", INTERNATIONAL JOURNAL OF NANOMEDICINE, vol. 12, 7 February 2017 (2017-02-07), pages 1033 - 1046, XP055799521

## Description

## Technical Field

The present disclosure relates to an oral pharmaceutical composition including teriparatide and a method for preparing the same. The present disclosure relates to an oral pharmaceutical composition including an ionic bond complex composed of teriparatide, deoxycholic acid, Nα-deoxycholyl-L-lysyl-methylester (DCK), and di-alpha-tocopherol polyethylene glycol 1000 succinate.

## Background Art

Parathyroid hormone (PTH) is a polypeptide composed of 84 amino acids secreted by the parathyroid glands and is one of the main hormones that regulate the concentration of calcium ions in the blood. Parathyroid hormone has anabolic (bone formation) and catabolic functions in the bone, and these actions depend on the duration and pattern of exposure to PTH. In addition, an anabolic function is promoted by intermittent PTH exposure and causes differentiation and proliferation of osteoblasts or reduction of apoptosis. A catabolic function is promoted by continuous PTH exposure and is associated with increased expression of receptor activator of nuclear factor-κB ligand (RANKL) and decreased expression of osteoprotegerin.

Teriparatide (PTH (1-34)), 34 amino acid residues from the N-terminus of PTH, is essential for the activation of type 1 PTH receptors, which are frequently expressed in bones and kidneys. When PTH (1-34) binds to the type 1 PTH receptor, a series of signaling systems, including the G protein-dependent cAMP/protein kinase A pathway, is activated, and calcium concentration is regulated. Teriparatide is a recombinant human PTH (1-34) (rhPTH (1-34)) and is clinically used to treat osteoporosis in postmenopausal women and osteoporosis in gonadotropic men. Intermittent administration of teriparatide stimulates new bone formation by activating more osteoblasts than osteoclasts and reduces the risk of fractures in osteoporosis. Many other osteoporosis treatments do not have catabolic properties and are prophylactic treatments that only inhibit the activity of osteoclasts. These antiresorptive include bisphosphonate, estrogen receptor modulator, and calcitonin. On the other hand, teriparatide may be used to treat osteoporosis in patients at risk of fracture and who do not respond to other bone resorption inhibitors.

Teriparatide is currently administered by subcutaneous injection into the thigh or abdomen once a day for 2 years. However, this method of subcutaneous injection causes pain to the patient and is accompanied by inconvenience in that the patient has to learn the correct injection method. Therefore, the development of alternative drug delivery systems such as oral administration, transdermal administration, and nasal administration for PTH peptides is being attempted in order to increase patient compliance. The advantage of oral administration is that the patient's convenience and the time it takes for the teriparatide concentration to reach $T_{max}$ increase. However, one of the biggest problems with the oral delivery of PTH is the low oral bioavailability of the peptide itself. Causes of these problems include a short half-life of the peptides, decomposition by gastric acid and digestive tract proteolytic enzymes, and low gastrointestinal cell membrane permeability due to the large molecular weight (about 4117.72 Da) of peptides. Therefore, developing a new administration delivery system for teriparatide to increase patient compliance is urgently required.

A technique for improving the above problems is disclosed in PCT/DK2004/000482 (published as WO2005002549A1). The technique of PCT/DK2004/000482 (published as WO2005002549A1) attempted to improve the absorption rate by minimizing the decomposition of drugs in the stomach by allowing PTH to be released 2 hours after oral administration with such as a normal intestinal coating. However, the low oral bioavailability of PTH is mainly due to the low intestinal membrane permeability itself due to the low lipid affinity and large molecular weight of the drug molecule, as well as the decomposition of the drug in the gastrointestinal tract. Therefore, a general enteric oral dosage form is insufficient to exhibit sufficient oral bioavailability to exert the therapeutic effect of PTH. In addition, in PCT/CZ2012/000025 (published as WO2012130193A1), teriparatide was prepared as a complex by non-covalent bonding with water-soluble polysaccharides such as beta-glucan, alginic acid, and chitosan to minimize decomposition of the drug enzyme to improve oral bioavailability. However, a method for improving intestinal membrane permeability thereof is not suggested. CN102125520A discloses an emulsion containing a hydrophilic biological macromolecule, a preparation method and application thereof, belonging to the fields of pharmacology and pharmaceutics. In particular, the emulsion contains a hydrophilic biological macromolecule, a water phase, an oil phase and an emulsifying agent. The emulsion is characterized by also comprising vitamin E and/or ester derivatives of vitamin E. The emulsion disclosed in CN102125520A has the characteristic of high oral bioavailability and is safe and effective.

On the other hand, in PCT/ IL2017/050920 (published as WO2018033927A1), the oral formulation of PTH (1-34) including NAC (8-N-(2-hydroxybenzoyl) aminocaprylate), NAD (10-N- (2-hydroxybenzoyl) aminodecanoic acid), 5-CNAC (8-N-(5-chlorosalicilyl) aminocaprylic acid), 4-MOAC (8-N-(2-hydroxy-4-methoxybenzoyl) aminocaprylic acid), 4-CNAB (4-N-(2-hydroxy-4-chlorobenzoyl) aminobutanoic acid) and their salt-like absorption enhancers are shown. The absorption enhancer used in the present disclosure is a synthetic surfactant and non-specifically increases the absorption rate of the drug in the gastrointestinal tract. Therefore, in the case of small animals, the absorption-enhancing effect of the drug can be exhibited even with a small amount of use, but when the volume of gastrointestinal fluid, such as primates and humans, is large, achieving sufficient absorption enhancement effect is difficult due to dilution of the absorption enhancer contained in the unit formulation by digestive fluid and water present in the intestine. In addition, there is a disadvantage that an excessive amount of an absorption enhancer must be taken in order to exhibit the same level of gastrointestinal absorption enhancing action as in small animals, which can cause unsaid side effects in the gastrointestinal tract by new synthetic surfactants.

Accordingly, the present inventors have made intensive research efforts to overcome the problems of the related art. As a result, a complex is formed with a drug and a non-covalent bond such as an ionic bond at the molecular level, and at the same time, when deoxycholic acid and deoxycholic acid derivatives are used as absorption enhancers to increase the absorption efficiency of drugs by specifically and selectively binding to bile acid carriers present in the intestinal cell membrane and TPGS is used as a solubilizing agent, it was confirmed that the absorption efficiency of oral drugs is increased, and then the present disclosure was completed.

## Disclosure

### Technical Problem

Accordingly, the main objective of the present disclosure is to provide an oral pharmaceutical composition including teriparatide, which can increase intestinal membrane permeability and oral administration bioavailability of teriparatide and improve patient compliance.

Another objective of the present disclosure is to provide a preparation method for preparing an oral pharmaceutical composition, including teriparatide.

[Korea National R&D Project that supported this disclosure]
[Project identification number] 10215797
[Ministry name] Ministry of SMEs and Startups, Republic of Korea
[Research management institution] Korea Carbon Convergence Technology Institute
[Research project name] Startup Leap Package
[Research project name] Drugs that enhance the activity of immunosuppressants using oral metronomic anticancer drugs Development
[Organization] ICURE BNP Co., Ltd.
[Korea national R&D project that supported this disclosure]
[Project unique number] P0010215
[Name of Ministry] Ministry of Trade, Industry and Energy of the Republic of Korea
[Research and management institution] Korea Institute for Advancement of Technology
[Research project name] Basic research rediscovery support
[Research project name] Development of anticancer drug platform technology through oral dosage form change
[Organization] ICURE BNP Co., Ltd.

### Technical Solution

According to one aspect of the present disclosure, the present disclosure provides an oral pharmaceutical composition including an ionic complex composed of teriparatide, deoxycholic acid, N$\alpha$-deoxycholyl-L-lysyl-methylester (DCK), and di-alpha-tocopherol polyethylene glycol 1000 succinate.

Teriparatide is a drug used for the treatment of osteoporosis and is currently administered by subcutaneous injection into the thigh or abdomen once a day for 2 years. However, this method of subcutaneous injection causes pain to the patient and is accompanied by inconvenience in that the patient has to learn the correct injection method. Accordingly, the inventors confirmed that when an oral pharmaceutical composition containing an ionic composite composed of teriparatide, deoxycholic acid, N$\alpha$-deoxycholyl-L-lysyl-methylester (DCK), which are deoxycholic acid derivatives, and di-alpha-tocopherol polyethylene glycol 1000 succinate are used, the intestinal membrane permeability and oral administration bioavailability of the drug can be increased and the patient's medication compliance can be improved, and the present disclosure has been completed.

In the oral pharmaceutical composition of the present disclosure, the teriparatide is a fragment of parathyroid hormone (PTH (1-34)), in addition to the PTH (1-34), PTH (1-28), the teriparatide may include at least one teriparatide selected from the group composed of PTH (1-31), PTH (1-38), and PTH (1-41).

Among the amino acid sequences constituting the peptide of teriparatide, there are a total of four amino acids of aspartic acid (Asp) and glutamic acid (Glu) that can bind to N$\alpha$-deoxycholyl-L-lysyl-methylester (DCK), which is a deoxycholic acid derivative with a positive charge. Since there is a total of 8 positively charged amino acids of arginine (Arg), histidine (His), and lysine that can bind negatively charged deoxycholic acid, up to 12 deoxycholic acid and deoxycholic acid per molecule of teriparatide derivatives can be ionically linked.

All sites of the teriparatide molecule available for binding to the deoxycholic acid molecule can be utilized to increase molecules of the deoxycholic acid and the deoxycholic acid derivative bonded to the teriparatide, thereby increasing the possibility that the deoxycholic acid and the deoxycholic acid derivatives may bond to bile acid transporters present in intestinal membrane cells. As a result, when the ionic bond composite, including the deoxycholic acid and the deoxycholic acid derivative, is used as the oral pharmaceutical composition, the intestinal membrane permeability and bioavailability of the teriparatide may be significantly improved.

In the oral pharmaceutical composition of this disclosure, the deoxycholic acid derivative N$\alpha$-deoxycholyl-L-lysyl-methylester (DCK) may be included in an amount of 0.1 to 10 moles, preferably 2 to 8 moles, based on 1 mole of teriparatide. When the deoxycholic acid derivative exceeds 10 moles, the absorption promotion effect of the drug is not rapidly increased compared to the amount in which the deoxycholic acid derivative is added, and a considerable amount of the non-bonded deoxycholic acid derivative may act as a surfactant, hence causing side effects such as irritation of the intestinal mucosa.

In the oral pharmaceutical composition of the present disclosure, the deoxycholic acid may be included in an amount of 1 to 20 moles, preferably 4 to 16 moles based on 1 mole of teriparatide. When the deoxycholic acid exceeds 20 mol, the absorption promotion effect of the drug is not significantly increased compared to the amount of deoxycholic acid added, and a considerable amount of non-bonded deoxycholic acid may serve as a surfactant, hence causing side effects such as mucosal irritation.

According to an embodiment of the present disclosure, as a result of confirming the intestinal membrane permeability of the ionic bond complex composed of teriparatide, deoxycholic acid, deoxycholic acid derivatives N$\alpha$-deoxycholyl-L-lysyl-methylester (DCK), and di-alpha-tocopherol polyethylene glycol 1000, according to the present disclosure (Example 1), it exhibited remarkably higher intestinal membrane permeability as compared to Comparative Examples 1 and 2 that did not contain a solubilizing agent. In addition, the intestinal membrane permeability was significantly higher than Comparative Examples 3, 5, and 6 using poloxamer, Labrasol, or Cremophor instead of TPGS as a solubilizing agent. In addition, it was confirmed that the intestinal membrane permeability of the Example in which TPGS was used alone was more improved than Comparative Example 4 in which TPGS and poloxamer were used together as a solubilizing agent. Furthermore, it was confirmed that the intestinal membrane permeability of the Example of the present disclosure in which N$\alpha$-deoxycholyl-L-lysyl-methylester (DCK) and deoxycholic acid were included together was more improved than Comparative Example 7 in which N$\alpha$-deoxycholyl-L-lysyl-methylester (DCK), which is an intestinal membrane permeation enhancer, was used alone.

From these results, the ionic bond complex, according to the present disclosure, can significantly improve intestinal membrane permeability when TPGS is used rather than other solubilizing agents. When deoxycholic acid, as well as deoxycholic acid derivatives are included in the ionic bond complex, the intestinal membrane permeability can be significantly improved because more ionic bonds are formed with teriparatide. In an oral pharmaceutical composition including teriparatide, it is suitable to include a deoxycholic acid derivative N$\alpha$-deoxycholyl-L-lysyl-methylester (DCK) and deoxycholic acid together in order to increase the intestinal membrane permeability of teriparatide, suggesting that TPGS is the most suitable solubilizing agent.

In the oral pharmaceutical composition of the present disclosure, the oral pharmaceutical composition may further include a poloxamer in addition to TPGS as a solubilizing agent, and in addition to the poloxamer, caprylocaproyl mqacrogol-8 glycerides (Labrasol) or Cremophor may be further included.

According to another aspect of the present disclosure, the present disclosure provides a method for preparing an oral pharmaceutical composition, the method including: a first step of forming an ionic complex by adding an aqueous solution of deoxycholic acid and N$\alpha$-deoxycholyl-L-lysyl-methylester (DCK) to the solution including teriparatide and di-alpha-tocopherol polyethylene glycol 1000 succinate (D-$\alpha$-tocopherol polyethylene glycol 1000 succinate); a second step of preparing granules by mixing a binder, a disintegrant, a diluent, and a lubricant with the ionic bond complex prepared in the first step; and a third step of compressing the granules prepared in the second step in the form of tablets.

In the method for preparing the pharmaceutical composition for oral use of the present disclosure, a binder, a disintegrant, a diluent, and a lubricant may be further included as in the second step in order to prepare the ionic bond complex into a tablet. As the binder, any binder conventionally used for manufacturing tablets may be used, for example, the binder may include at least one selected from the group composed of polyvinyl pyrrolidone, gelatin, starch, sucrose, methyl cellulose, ethyl cellulose, hydroxypropyl cellulose, hydroxypropyl alkyl cellulose. In addition, as the disintegrant, any disintegrant conventionally used for manufacturing tablets may be used, for example, the disintegrant may include at least one selected from the group composed of cross-linked polyvinyl pyrrolidone, cross-linked sodium carboxymethyl cellulose, cross-linked calcium carboxymethyl cellulose, cross-linked carboxymethyl cellulose, sodium starch glycolate, carboxymethyl starch, sodium carboxymethyl starch, potassium methacrylate-divinyl benzene copolymer, amylose, cross-linked amylose, a starch derivative, microcrystalline cellulose, cellulose derivative, cyclodextrin, and dextrin derivatives, As the diluent, any diluent conventionally used for preparing tablets may be used, for example, the diluent may include at least one selected from the group composed of lactose, dextrin, starch, microcrystalline cellulose, calcium hydrogen phosphate, anhydrous calcium hydrogen phosphate, calcium carbonate, and saccharides. As the lubricant, any lubricant conventionally used for preparing tablets may be used, for example, the lubricant may include at least one selected from the group composed of stearic acid, zinc stearate, magnesium stearate, calcium stearate, and talc.

In the oral pharmaceutical composition of the present disclosure, the composition further includes the step of coating the tablet prepared in the third step with an enteric material. By forming a coating layer on the surface of the tablet prepared in the third step, it is possible to minimize the decomposition of the drug by inhibiting the release of the drug under acidic conditions in the stomach after oral administration.

In the oral pharmaceutical composition of the present disclosure, as the enteric material, any enteric material conventionally used for preparing tablets may be used, for example, the enteric material may include at least one selected from the group composed of methacrylic acid-ethyl acrylate copolymer (Eudragit), hydroxypropyl methylcellulose phthalates, acetyl succinate hydroxyl propyl methylcellulose, cellulose acetate phthalate, polyvinyl acetate phthalates, carboxymethyl ethyl cellulose, and shellac.

The enteric coating layer may further include a plasticizer, and in addition, a pigment, antioxidant, talc, titanium dioxide, flavoring agent, etc. As the plasticizer, the plasticizer may use one or more components selected from the group composed of castor oil, fatty acids, substituted triglycerides and glycerides, and polyethylene glycol having a molecular weight of 300 to 50,000, and derivatives thereof. The solvent of the coating solution for preparing the enteric coating layer may be water or an organic solvent, and as the organic solvent, ethanol, isopropanol, acetone, chloroform, dichloromethane, or a mixture thereof may be used.

According to another aspect of the present disclosure, the present disclosure provides a method for preparing an oral pharmaceutical composition, the method including: the first step of forming an ionic bond complex by adding an aqueous solution of deoxycholic acid and N$\alpha$-deoxycholyl-L-lysyl-methylester (DCK) to the solution including teriparatide and di-alpha-tocopherol polyethylene glycol 1000 succinate (D-$\alpha$-tocopherol polyethylene glycol 1000 succinate); a second step of preparing a mixture by adding caprylocaproyl mqacrogol-8 glycerides (Labrasol) as a primary surfactant and Tween 80 as a primary auxiliary surfactant to the ionic bond complex solution; a third step of preparing a water-in-oil (w/o) first nano-emulsion by dispersing the mixture of the second step on a primary oil phase; and a fourth step of preparing a water-in-oil-in-water (w/o/w) secondary nano-emulsion by adding a mixture of Cremopore or Twin 80 as a secondary surfactant and polyethylene glycol 400 as a secondary auxiliary surfactant to the water-in-oil (w/o) first nano-emulsion of the third step.

In the method for preparing the oral pharmaceutical composition of the present disclosure, the primary oil phase is at least one selected from the group composed of silicone oil, ester oil, hydrocarbon-based oil, propylene glycol monocaprylate, propylene glycol dicaprylocaprate, oleoyl macrogol-6 glycerides, lauroyl macrogol-6 glycerides, linoleic oil macrogol-6 glycerides, medium chain triglycerides, oleic acid, stearic acid, glyceryl behenate, glycerol monostearate, and castor oil.

In the method for preparing the oral pharmaceutical composition of the present disclosure, the primary oil phase in the w/o/w secondary nano-emulsion may be included in an amount of 0.1 to 40% by weight, preferably 1 to 20% by weight, based on the total weight of the composition. When the oil is less than 0.1% by weight, it is difficult to disperse the aqueous ionic bond complex solution in the oil phase, and when it exceeds 40% by weight, it is not dispersed in the secondary external aqueous phase, and phase separation may occur.

In the method for preparing the oral pharmaceutical composition of the present disclosure, the mixture of the primary surfactant and the primary auxiliary surfactant and the mixture of the secondary surfactant and the secondary auxiliary surfactant are included in an amount of 0.1 to 40% by weight based on the total weight of the composition. When the surfactant and auxiliary surfactant mixture is included in an amount of less than 0.1% by weight, it is difficult to disperse the primary internal aqueous phase in the primary oil phase, and when the mixture of the secondary surfactant and the secondary auxiliary surfactant exceeds 40% by weight, the weight of the surfactant, oil, and the internal aqueous phase is relatively reduced, as a result, a problem of phase separation may occur.

The primary and secondary surfactants used in the present disclosure allow the aqueous phase in which the teriparatide complex is dispersed to be dispersed in the oil phase, and after oral administration, the w/o nano-emulsion is dispersed in gastric fluids and intestinal fluids in the gastrointestinal tract.

Surfactants used in the preparation of nano-emulsions may be at least one selected from the group composed of poloxamer, caprylocaproyl mqacrogol-8 glycerides (Labrasol), Cremophor, glycerol monocaprylocaphrate (Capmul MCM), lauroyl macrogol-32 glycerides (Gelucire 44/14), Solutrol, polysorbate (Tween), sorbitan monolaurate (Span), and mixtures thereof.

In the method for preparing the oral pharmaceutical composition of the present disclosure, di-alpha-tocopherol polyethylene glycol 1000 succinate is included in an amount of 0.1 to 100 parts by weight based on 1 part by weight of teriparatide.

The primary and secondary auxiliary surfactants that can be used in the present disclosure allow the surface energy to be reduced so that the inner aqueous phase in which the active material teriparatide complex is dispersed can be dispersed in the oil phase by these surfactants. After oral administration, the primary and secondary auxiliary surfactants allow the water-in-oil (w/o) nano-emulsion to be well dispersed in gastric and intestinal fluids in the gastrointestinal tract.

Auxiliary surfactants used in the preparation of nano-emulsions may be at least one selected from the group composed of diethylene glycol monoethylether (Transcutol HP), polysorbate, polyethylene glycol, butylene glycol), propylene glycol, ethanol, isopropanol, and mixtures thereof. In this case, the auxiliary surfactant may be mixed with the surfactant, and a mixing ratio of the auxiliary surfactant to the surfactant may be 1:0.1 to 1:10 by weight, preferably 1:0.5 to 1:2 by weight. In addition, the auxiliary surfactant may be included in an amount of 0.1 to 40% by weight, preferably 1 to 20% by weight, based on the total weight of the composition.

The water-in-oil-in-water (w/o/w) nano-emulsion prepared according to the above preparing method may be prepared by being filled in a soft or hard capsule and may further include coating the obtained capsule with a coating solution, including an enteric coating agent.

**Advantageous Effects**

As described above, the oral pharmaceutical composition including teriparatide according to the present disclosure is prepared by using deoxycholic acid, $N\alpha$-deoxycholyl-L-lysyl-methylester (DCK), and TPGS for teriparatide, an osteoporosis treatment agent, to prepare an ionic bond complex and is prepared in tablet form or supported in a water-in-oil-in-water nano-emulsion. The oral pharmaceutical composition can improve intestinal membrane permeability and bioavailability and can improve patient compliance.

**Description of Drawings**

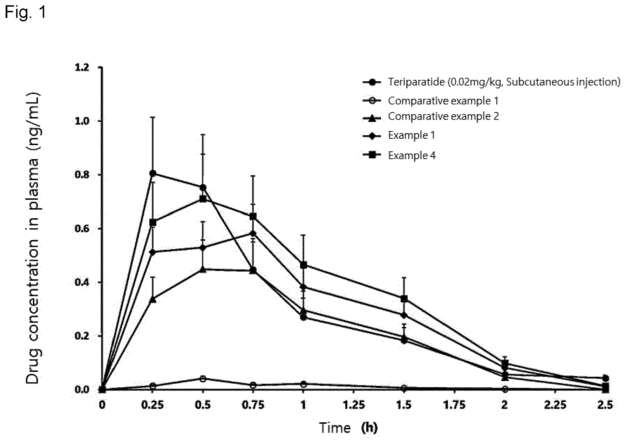

FIG. 1 shows the results of confirming the bioavailability of teriparatide according to Examples 1 and 4 of the present disclosure.

**Best Mode for Disclosure**

Hereinafter, the present disclosure will be described in more detail through examples. These examples are only for showing the present disclosure.

Preparation Example: Preparation of deoxycholic acid derivatives

Deoxycholic acid derivatives were prepared by chemically binding positively charged lysine to deoxycholic acid. First, 26 g of deoxycholic acid is dissolved in 800 mL of tetrahydrofuran. Separately 20 g of HLys(Boc)-OMe.HCl is dissolved in a mixed solvent of 7.4 mL of N-methyl morpholine and 6.4 mL of ethyl chloroformate. An HLys(Boc)-OMe·HCl solution was added to the deoxycholic acid solution, followed by stirring for 30 minutes, and then refluxed for 2 hours. The reaction precipitate obtained by stirring at room temperature overnight was filtered, and then the residual solvent was evaporated. The dried precipitate is purified through column chromatography using chloroform and methanol and then dissolved in a mixed solvent of acetyl chloride and methanol in a cooling water bath (ich batch). After removing the solvent, the residue was dissolved in water again, washed three times with chloroform, and then a water layer was taken and freeze-dried to prepare $N\alpha$-deoxycholyl-L-lysyl-methylester (DCK), which is a deoxycholic acid derivative.

Example 1 and Comparative Example: Preparation of ionic bond complexes of teriparatide, deoxycholic acid, deoxycholic acid derivatives, and solubilizing agent

After dissolving teriparatide and di-alpha-tocopherol polyethylene glycol 1000 succinate (TPGS) as a solubilizer in purified water, an aqueous solution of a deoxycholic acid derivative prepared separately is slowly added while stirring to prepare an ionic bond complex. At this time, the deoxycholic acid derivative aqueous solution is slowly added so that the molar ratio of teriparatide and the deoxycholic acid derivative, $N\alpha$-deoxycholyl-L-lysyl-methylester (DCK) is 1:2 or 1:4. Then, an ionic bond complex is prepared by slowly adding an aqueous sodium deoxycholic acid solution prepared separately while stirring the complex solution. At this time, an aqueous solution of deoxycholic acid is slowly added so that the molar ratio of teriparatide and deoxycholic acid is 1:4 or 1:8. The final mixture was centrifuged and then freeze-dried to prepare powdery teriparatide and deoxycholic acid derivatives, teriparatide and deoxycholic acid, or teriparatide, and a mixture composite thereof by the composition of Table 1 below.

In addition, as a Comparative Example, the complex was prepared without deoxycholic acid using poloxamer, caprylocaproyl macrogol-8 glycerides (trade name: Labrasol), and Cremophor as a solubilizing agent.

[Table 1]

| Ingredient name | Example 1 | Comparative example 1 | Comparative example 2 | Comparative example 3 | Comparative example 4 | Comparative example 5 | Comparative example 6 | Comparative example 7 |
|---|---|---|---|---|---|---|---|---|
| Teriparatide | 0.1 | 0.1 | 0.1 | 0.1 | 0.1 | 0.1 | 0.1 | 0.1 |
| Deoxycholic acid derivative | 0.056 | - | 0.056 | 0.056 | 0.056 | 0.056 | 0.056 | 0.056 |
| Deoxycholic acid | 0.08 | - | - | - | - | - | - | - |
| TPGS | 7.52 | - | - | - | 7.52 | - | - | 7.52 |
| Poloxamer | - | - | - | 10 | 10 | - | - | - |
| Labrasol | - | - | - | - | - | 7.52 | - | - |
| Cremophor | - | - | - | - | - | - | 7.52 | - |
| Total weight (mg) | 7.76 | 0.1 | 1.056 | 10.056 | 17.676 | 7.676 | 7.676 | 7.676 |

Experimental Example 1: Confirmation of the artificial intestinal membrane permeability of the complex composed of teriparatide, deoxycholic acid, deoxycholic acid derivatives, and solubilizing agents

Effective permeability (Pe) through the artificial intestinal membrane of Examples 1 and Comparative Examples 1 to 7 prepared above was evaluated using the parallel artificial membrane permeability assay (PAMPA), which is an artificial intestinal membrane permeability evaluation system. First, the samples of Example 1 and Comparative Examples 1 to 7 were dissolved in phosphate buffer (PBS, pH 6.8) to a concentration of 200 ug/mL as teriparatide, and then 200 $\mu$L each was added to the donor part of the PAMPA system, and 300 $\mu$L of phosphate buffer (PBS, pH 6.8) was filled in the receiving part of the PAMPA system. Thereafter, the donor part and the receiving part were combined and left at room temperature for 5 hours. Then, the solution of each well of the receiving part and the donor part was filtered through a membrane filter having a pore diameter of 0.45 $\mu$m, and then the concentration of teriparatide permeated through the artificial intestinal membrane was analyzed using the HPLC system under the following conditions.

50 $\mu$L of each sample was injected into the HPLC system, and acetonitrile (B) containing 0.1% (w/v) trifluoroacetic acid (TFA) aqueous solution (A) and 0.1% (w/v) TFA as mobile phases were separated by linearly changing the composition of mobile phase B from 51% to 76% for 25 minutes at a flow rate of 1.0 mL/min. Teriparatide was measured at 220 nm, and the effective transmittance (Pe) through the artificial intestinal membrane was calculated by Formula 1 below.

[Formula 1]

$$P_e = -\ln[1 - C_R(t)/C_{equilibrium}]/[S \times (1/V_D + 1/V_R) \times t]$$

Where Pe is the effective transmittance (cm/s), S is the effective permeation area (0.288 cm$^2$), VD is the solution volume of the donor part well (0.2 mL), VR is the solution volume of the receiving part well (0.3 mL), t is the sampling time (s), CR(t) is the drug concentration in the receiving part at time t, C$_{equilibrium}$ is [CD(t) $\times$ VD CR(t) $\times$ VR]/(VD+VR), and CD(t) is the drug concentration at the donor part.

The results of calculating the effective transmittance through the artificial intestinal membrane of Example 1 and Comparative Examples 1 to 7 using the above Formula are shown in Table 2 below.

[Table 2]

|  | Effective transmittance ($P_e$, x $10^{-6}$cm/s) |
| --- | --- |
| Example 1 | 14.9 ± 4.79 |
| Comparative example 1 | 0.582 ± 0.132 |
| Comparative example 2 | 2.36 ± 0.646 |
| Comparative example 3 | 7.57 ± 2.09 |
| Comparative example 4 | 11.9 ± 3.38 |
| Comparative example 5 | 8.27 ± 2.47 |
| Comparative example 6 | 6.52 ± 1.27 |
| Comparative example 7 | 9.07 ± 3.39 |
| *Each value means mean ± standard deviation (n = 4). | |

As a result, as can be seen in Table 2 above, in the case of Comparative Example 2, in which a complex of the deoxycholic acid derivative to teriparatide was formed in a ratio of 1:4 mol, compared to Comparative Example 1, teriparatide permeability through the artificial intestinal membrane increased by 4.05 times. This is considered to be due to the fact that the deoxycholic acid derivative itself increased the lipid solubility of the teriparatide and improved lipid permeability to the intestinal membrane.

However, when a solubilizing agent such as TPGS, poloxamer, caprylocaproyl mqacrogol-8 glycerides (Labrasol), and Cremophor is added during complex formation of teriparatide and a deoxycholic acid derivative, teriparatide permeability increases 3.84 times, 3.21 times, 3.50 times, and 2.76 times, respectively, compared to the permeability of the 1:4 molar ratio complex of the teriparatide and the deoxycholic acid derivative. In particular, in the case of a complex using TPGS as a solubilizing agent as in Example 1, the artificial intestinal membrane permeability increased by 25.6 times and 6.31 times, respectively, compared to teriparatide (Comparative Example 1) and a 1:4 molar ratio complex of teriparatide and deoxycholic acid derivatives (Comparative Example 2).

In addition, in the case of the complex using TPGS as a solubilizing agent as in Example 1, the artificial intestinal membrane permeability was increased compared to Comparative Examples 3, 5, and 6 using poloxamer, Labrasol, and Cremophor as the solubilizing agent.

Moreover, the artificial intestinal membrane permeability of Example 1 of the present disclosure using TPGS alone was increased compared to Comparative Example 4 in which TPGS and poloxamer were used together as a solubilizing agent.

In addition, the artificial intestinal membrane permeability of Example 1 of the present disclosure containing both deoxycholic acid and deoxycholic acid derivatives was increased compared to Comparative Example 7 containing deoxycholic acid derivatives Nα-deoxycholyl-L-lysyl-methylester (DCK) and TPGS without deoxycholic acid.

These results are considered to be due to the lipid affinity enhancing effect of the deoxycholic acid derivative itself and the solubilizing action of the intestinal lipid membrane by the solubilizing agent, in particular, the lipid affinity enhancing effect of teriparatide according to the intestinal lipid membrane solubilizing action appears to be due to the combination of deoxycholic acid and deoxycholic acid derivatives and TPGS.

Experimental Example 2: Confirmation of permeability through intestinal cell membrane of the complex composed of teriparatide, deoxycholic acid, deoxycholic acid derivatives, and solubilizing agent

The apparent permeability of the complexes prepared as in Example 1 and Comparative Examples 1 to 7 to the intestinal cell membrane, Caco-2 cell membrane, was evaluated as follows. After Caco-2 cells were treated at a concentration of 1×105 cells/mL in 24-well Transwell, respectively, and after culturing the cells for 14 to 16 days, a cell monolayer of the electrical resistance (TEER) value through the Caco-2 cell membrane was >350 Ω.cm$^2$ was used for the experiment. First, the medium was removed from the Transwell, and then the donor part and receiving part were filled with HBSS and cultured at 37°C for 20 minutes, then the TEER value was measured again, and then HBSS was removed. Thereafter, 0.1 mL of the drug solution in which the samples of Examples 1 and Comparative Examples 1 to 7 were dissolved at 200 μM as teriparatide in HBSS was applied to the donor part of each Transwell, and the receiving part was filled with 0.6 mL of HBSS and cultured at 37°C. At 0.5, 1, 2, 3, 4, and 5 hours, 100 μL samples were collected from the receiving part, filtered using a membrane filter (pore 0.45 um, PVDF), and the concentration of teriparatide permeated through the intestinal cell membrane was analyzed by HPLC under the conditions mentioned in Experimental Example 1. Apparent intestinal cell membrane permeability (Papp) was calculated using Formula 2 below, and the results are shown in Table 3.

[Formula 2]

$$P_{app} = dQ/dt \times 1/(S \times C_i)$$

Where $dQ/dt$ means the permeation rate (pmol/h) of the drug to the donor part, and S means the permeation area ($cm^2$). $C_i$ means the initial concentration ($\mu M$) of the drug at the donor part.

[Table 3]

| | Apparent transmittance ($P_{app}$, x $10^{-6}$ cm/s) |
|---|---|
| Example 1 | 4.70 ± 0.589 |
| Comparative example 1 | 0.362 ± 0.045 |
| Comparative example 2 | 1.04 ± 0.241 |
| Comparative example 3 | 2.01 ± 0.517 |
| Comparative example 4 | 3.56 ± 0.610 |
| Comparative example 5 | 1.90 ± 0.486 |
| Comparative example 6 | 1.68 ± 0.408 |
| Comparative example 7 | 3.13 ± 0.467 |
| * Each value means mean ± standard deviation (n = 4). | |

As a result, as shown in Table 3 above, the permeability through the intestinal cell membrane of the complex formed of teriparatide and deoxycholic acid derivative in a molar ratio of 1:4 (Comparative Example 2) was increased by 2.87 times compared to the teriparatide (Comparative Example 1). This effect of increasing intestinal membrane permeability of drugs by deoxycholic acid derivatives is due to the improvement of lipophilicity of drugs as well as the enhancement of cell membrane permeability of drugs resulting from selective and specific binding of deoxycholic acid to bile acid carriers present on the surface of the intestinal cell membrane.

However, in the deoxycholic acid derivative molecule bonded to a molecule of hydrophilic teriparatide peptide, the complex molecule itself forms a self-assembled micelle due to hydrophobicity so that the deoxycholic acid derivative molecule is located in the inner core of the micelle, which degrades specific interaction with the bile acid carrier present in the intestinal cell membrane. Therefore, in order to inhibit the formation of self-assembled micelles of such complexes, when solubilizing agents such as TPGS, poloxamer, caprylocaproyl mqacrogol-8 glycerides (Labrasol), and Cremopores are added in the ionic bond complex preparation process of the teriparatide-deoxycholic acid derivatives as in Example 1 and Comparative Examples 3 to 7, the intestinal cell membrane permeability was increased by 3.01 times, 1.93 times, 1.83 times, and 1.62 times, respectively, compared to the complex of teriparatide-deoxycholic acid derivative (Comparative Example 2) due to the increase in specific interactions of the deoxycholic acid derivative with the bile acid carriers and the synergistic effect of improving the solubility of the solubilizer itself. In particular, in the case of a complex using TPGS as a solubilizing agent as in Example 1, the artificial intestinal membrane permeability was increased by 13.0 times and 4.52 times, respectively, compared to teriparatide (Comparative Example 1) and a 1:4 molar ratio complex of teriparatide and deoxycholic acid derivatives (Comparative Example 2).

In addition, in the case of the complex using TPGS as a solubilizing agent as in Example 1, the intestinal cell membrane permeability was increased compared to Comparative Examples 3, 5, and 6 using poloxamer, Labrasol, and Cremophor as the solubilizing agent.

Moreover, the intestinal cell membrane permeability of Example 1 of the present disclosure using TPGS alone was increased compared to Comparative Example 4 in which TPGS and poloxamer were used together as a solubilizing agent.

In addition, the artificial intestinal membrane permeability of Example 1 of the present disclosure containing both deoxycholic acid and deoxycholic acid derivatives was increased compared to Comparative Example 7 containing deoxycholic acid derivatives Nα-deoxycholyl-L-lysyl-methylester (DCK) and TPGS without deoxycholic acid.

These results are considered to be due to the lipid affinity enhancing effect of the deoxycholic acid derivative itself and the solubilizing action of the intestinal lipid membrane by the solubilizing agent, in particular, the lipid affinity enhancing effect of teriparatide according to the intestinal lipid membrane solubilizing action appears to be due to the combination of deoxycholic acid and deoxycholic acid derivatives and TPGS.

Example 2: Preparation of an oral solid preparation including a complex composed of teriparatide, deoxycholic acid, a deoxycholic acid derivative, and a solubilizing agent

The complex composed of teriparatide, deoxycholic acid, deoxycholic acid derivative, and TPGS prepared in Example 1 was mixed with other additives shown in Table 4 below and subjected to a wet granulation process. The prepared granules were dried, mixed with magnesium stearate, and compressed into an appropriate form to prepare tablets or filled in hard capsules. The composition of the obtained matrix tablet and capsule contents are shown in Table 4 below.

[Table 4]

| Ingredient (mg) | Example 2 |
| --- | --- |
| Teriparatide | 0.1 |
| Deoxycholic acid derivatives | 0.056 |
| Deoxycholic acid | 0.08 |
| TPGS | 7.52 |
| Polyvinylpyrrolidone | 12.48 |
| Cross-linked sodium carboxymethylcellulose | 10 |
| Microcrystalline Cellulose | 34.382 |
| Lactose | 34.382 |
| Magnesium stearate | 1 |
| Moisture* | Appropriate amount |
| Total amount | 100 |
| * Removed during manufacturing. | |

Example 3: Coating of matrix tablets containing teriparatide complex

The matrix tablet prepared in Example 2 was primarily coated with hydroxypropyl methyl cellulose 2910 and then secondarily coated with hydroxypropyl methyl cellulose phthalate, which is an enteric coating agent containing a pigment, to prepare the tablet of Example 3. At this time, the composition of the coating solution is shown in Table 5 below, and the coating was spray-coated with a pan coater.

[Table 5]

| Division | Ingredient | Example 3 |
| --- | --- | --- |
| Primary coating | Hydroxypropyl methyl cellulose 2910 | 5* |
| Primary coating | Triethyl citrate | 0.5* |
| Secondary enteric coating | Hydroxypropyl methyl cellulose phthalate | 12* |
| * The ratio of the coating to the uncoated core matrix tablet is expressed in % by weight. | | |

Example 4: Preparation of oral water-in-oil-in-water type (w/o/w) nano-emulsion containing complex composed of teriparatide, deoxycholic acid, deoxycholic acid derivative, and solubilizing agent

After redispersing 17.76 mg of the complex of the teriparatide, deoxycholic acid, the deoxycholic acid derivative, and the TPGS (corresponding to 0.1 mg of teriparatide) prepared in Example 1 in 38 mg of purified water, 112 mg of 1:1 mixture of a primary surfactant and a primary auxiliary surfactant (Labrasol: Tween 80 = 1:1, w/w) was added and mixed, and then 50 mg of Labrafil M1944 in an oil phase was added and mixed to prepare a w/o nano-emulsion. A liquid oral nano-emulsion was prepared by adding 630 mg of a mixture of secondary surfactant and secondary auxiliary surfactant (Tween 80: Cremophor EL: PEG400 = 1:2:2, w/w/w).

Experimental Example 3: Confirmation of rat bioavailability of oral formulations

After anesthesia by intraperitoneal injection of ketamine (45mg/kg) and xylazine (5mg/kg) to female Sprague-Dawley rats (200-250g, 6-7 weeks old), the abdomen of the rat was cut to take out the small intestine, and Examples 1 and 4 and Comparative Examples 1 and 2 prepared above were dispersed in purified water, and then injected into a proximal jejunum in an amount corresponding to 100 μg/kg as teriparatide by 400 μL. In addition, in order to evaluate the relative bioavailability, 150 μL of teriparatide solution dissolved in physiological saline was subcutaneously injected at an amount corresponding to 20 ug/kg as teriparatide.

After drug administration, blood samples were collected 150 μL at regular time intervals and mixed with 3.8% aqueous sodium citrate solution of 50 μL. Thereafter, the blood sample was centrifuged for 15 minutes at 2,500 xg, at 4°C, and plasma was collected and stored at -70°C. The teriparatide concentration in plasma was measured at a wavelength of 620 nm using the human PTH (1-34) ELISA kit (ALPCO Diagnostics, USA). The pharmacokinetic parameters are estimated through the non-compartment method using WinNonlin S Software (ver. 5.3; Pharsight Corporation, USA) and are shown in Table 6 and FIG. 1.

[Table 6]

| Substance to be administered | Teriparati de | Comparati ve example 1 | Comparati ve example 2 | Examp le 1 | Examp le 4 |
|---|---|---|---|---|---|
| Route of administration | Subcutaneo us injection | Dosing in jejunum | Dosing in jejunum | Dosin g in jejun um | Dosin g in jejun um |
| Dosage as teriparatide (mg/kg) | 0.02 | 0.1 | 0.1 | 0.1 | 0.1 |
| $T_{max}{}^a$(h) | 0.25 ± 0.00 | 0.33 ± 0.14 | 0.42 ± 0.14 | 0.50 + 0.25 | 0.58 + 0.14 |
| $C_{max}{}^b$(ng/mL) | 0.805 ± 0.209 | 0.041 ± 0.010 | 0.449 ± 0.108 | 0.583 + 0.106 | 0.710 + 0.165 |
| $AUC_{last}{}^c$(ng .)sh/ mL) | 0.734 ± 0.191 | 0.030 ± 0.007 | 0.528 ± 0.128 | 0.732 + 0.133 | 0.893 + 0.208 |
| $AUC_{inf}{}^d$(ng .)shim L) | 0.765 ± 0.199 | 0.031 ± 0.007 | 0.556 ± 0.134 | 0.736 + 0.134 | 0.897 + 0.209 |
| Bioavailability[e] (%) | 100 | 0.810 ± 0.191 | 14.4 ± 3.48 | 20.0 + 3.63 | 24.3 + 5.66 |

[a]$T_{max}$, time to reach Cmax

[b]$C_{max}$, maximum plasma concentration

[c]$AUC_{last}$, the area under the plasma concentration-time curve from 0 to the last plasma concentration measurement time

[d]$AUC_{inf}$, plasma concentration from 0 to infinity - area under the time curve

[e]Bioavailability, ($AUC_{last}$, jejunum/dose teriparatide, jejunum) / ($AUC_{last}$, subcutaneous injection/dose teriparatide, subcutaneous injection) × 100

Each value means mean ± standard deviation (n = 4).

FIG. 1 shows the concentration of teriparatide in plasma over time after administration of a drug to a rat. After administration of 0.1 mg/kg teriparatide of Comparative Example 1 intrajejunal, the maximum drug concentration ($C_{max}$) in plasma was 0.041 ± 0.010 ng/mL, the area under the time-concentration curve ($AUC_{last}$) was 0.030 ± 0.007 ng.h/mL, and the relative bioavailability compared to subcutaneous injection was evaluated to be 0.810 ± 0.191%. On the other hand, when comparing the case where 0.1 mg/kg as teriparatide of the 1:4 molar complex of the teriparatide-deoxycholic acid derivative (Comparative Example 2) was administered in the jejunum and the case where 0.1 mg/kg of teriparatide alone was administered in the jejunum, in case of a complex of the teriparatide-deoxycholic acid derivative, $C_{max}$ was 11 times, $AUC_{last}$ was 17.6 times, and relative bioavailability compared to subcutaneous injection was increased by 17.8 times. On the other hand, when the complex of teriparatide, deoxycholic acid, a deoxycholic acid derivative, and TPGS of Example 1 was administered, compared to which Comparative Example 1 (teriparatide alone) or Comparative Example 2 (teriparatide and deoxycholic acid derivative 1 : 4 molar complex) was administered, respectively, $C_{max}$ was increased by 14.2 times and 1.30 times, $AUC_{last}$ was increased by 24.4 times and 1.39 times, respectively, and relative bioavailability compared to subcutaneous injection increased by 24.7 times and 1.39 times, respectively. In the case of the oral nano-emulsion of Example 4, including a complex of teriparatide, deoxycholic acid, a deoxycholic acid derivative, and TPGS of Example 1, $C_{max}$ and $AUC_{last}$ were increased by 17.3 times and 29.8 times, respectively, compared to Comparative Example 1 (teriparatide alone) and the relative bioavailability compared to subcutaneous injection was evaluated as $24.3 \pm 5.66\%$, increasing by 30 times.

**Claims**

1. An oral pharmaceutical composition comprising an ionic bond complex composed of teriparatide, deoxycholic acid, N$\alpha$-deoxycholyl-L-lysyl-methylester, and di-alpha-tocopherol polyethylene glycol 1000 succinate.

2. The composition of claim 1, wherein the N$\alpha$-deoxycholyl-L-lysyl-methylester is contained in 0.1 to 10 moles based on 1 mole of teriparatide.

3. The composition of claim 1, wherein the deoxycholic acid is contained in 1 to 20 moles based on 1 mole of teriparatide.

4. The composition of claim 1, further comprising a poloxamer.

5. A method for preparing an oral pharmaceutical composition, the method comprising:

   a first step of forming an ionic complex by adding an aqueous solution of deoxycholic acid and N$\alpha$-deoxycholyl-L-lysyl-methylester to a solution including teriparatide and di-alpha-tocopherol polyethylene glycol 1000 succinate;
   a second step of preparing granules by mixing a binder, a disintegrant, a diluent, and a lubricant with the ionic bond complex prepared in the first step; and
   a third step of compressing the granules prepared in the second step in the form of tablets.

6. The method of claim 5, further comprising a step of coating the tablet prepared in the third step with an enteric material.

7. The method of claim 6, wherein the enteric material is at least one selected from the group composed of methacrylic acid-ethyl acrylate copolymer (Eudragit), hydroxypropyl methylcellulose phthalate, acetyl succinate hydroxypropyl methylcellulose, cellulose acetate phthalate, polyvinyl acetate phthalate, carboxymethyl ethyl cellulose, and shellac.

8. A method for preparing an oral pharmaceutical composition, the method comprising:

   a first step of forming an ionic complex by adding an aqueous solution of deoxycholic acid and N$\alpha$-deoxycholyl-L-lysyl-methylester to a solution including teriparatide and di-alpha-tocopherol polyethylene glycol 1000 succinate;
   a second step of preparing a mixture by adding caprylocaproyl mqacrogol-8 glycerides as a primary surfactant and Tween 80 as a primary auxiliary surfactant to the ionic bond complex solution;
   a third step of preparing a water-in-oil (w/o) primary nano-emulsion by dispersing the mixture of the second step on a primary oil phase; and
   a fourth step of preparing a water-in-oil-in-water (w/o/w) secondary nano-emulsion by adding a mixture of Cremopore or Twin 80 as a secondary surfactant and polyethylene glycol 400 as a secondary auxiliary surfactant to the water-in-oil (w/o) primary nano-emulsion of the third step.

9. The method of claim 8, wherein the primary oil phase is at least one selected from the group composed of silicone oil, ester-based oil, hydrocarbon-based oil, propylene glycol monocaprylate, propylene glycol dicaprylocaprate, oleoyl macrogol-6 glycerides, lauroyl macrogol-6 glycerides, linoleic oil macrogol-6 glycerides, medium-chain triglycerides, oleic acid, stearic acid, glyceryl behenate, glycerol monostearate, and castor oil.

10. The method of claim 8, wherein the primary oil phase in the w/o/w secondary nano-emulsion is contained in an amount of 0.1 to 40% by weight based on the total weight of the composition.

11. The method of claim 8, wherein the mixture of the primary surfactant and the primary auxiliary surfactant and the mixture of the secondary surfactant and the secondary auxiliary surfactant are contained in an amount of 0.1 to 40% by weight based on the total weight of the composition.

12. The method of claim 8, wherein the primary and secondary auxiliary surfactants are mixed with the primary and secondary surfactants, respectively, each independently in a weight ratio of 1:0.1 to 1:10.

13. The method of claim 8, wherein the di-alpha-tocopherol polyethylene glycol 1000 succinate is contained in an amount of 0.1 to 100 parts per 1 part by weight of teriparatide.

**Patentansprüche**

1. Eine orale pharmazeutische Zusammensetzung, die einen Ionenbindungskomplex aus Teriparatid, Desoxycholsäure, Nα-Desoxycholyl-L-lysyl-methylester und Di-alpha-Tocopherolpolyethylenglykol-1000-succinat enthält.

2. Zusammensetzung nach Anspruch 1, wobei der Nα-Desoxycholyl-L-lysylmethylester in 0,1 bis 10 Mol, bezogen auf 1 Mol Teriparatid, enthalten ist.

3. Zusammensetzung nach Anspruch 1, wobei die Desoxycholsäure in 1 bis 20 Mol, bezogen auf 1 Mol Teriparatid, enthalten ist.

4. Zusammensetzung nach Anspruch 1, die außerdem ein Poloxamer umfasst.

5. Ein Verfahren zur Herstellung einer oralen pharmazeutischen Zusammensetzung, wobei das Verfahren umfasst: ein erster Schritt der Bildung eines ionischen Komplexes durch Zugabe einer wässrigen Lösung von Desoxycholsäure und Nα-Desoxycholyl-L-lysyl-methylester zu einer Lösung, die Teriparatid und Di-alpha-Tocopherolpolyethylenglykol-1000-succinat enthält;

   einen zweiten Schritt der Herstellung von Granulaten durch Mischen eines Bindemittels, eines Sprengmittels, eines Verdünnungsmittels und eines Schmiermittels mit dem im ersten Schritt hergestellten Ionenbindungskomplex;
   und
   einen dritten Schritt des Verpressens des im zweiten Schritt hergestellten Granulats in Form von Tabletten.

6. Das Verfahren gemäß Anspruch 5, das außerdem einen Schritt des Beschichtens der im dritten Schritt hergestellten Tablette mit einem magensaftresistenten Material umfasst.

7. Verfahren nach Anspruch 6, wobei das magensaftresistente Material mindestens eines ist, ausgewählt aus der Gruppe bestehend aus Methacrylsäure-Ethylacrylat-Copolymer (Eudragit), Hydroxypropylmethylcellulosephthalat, Acetylsuccinathydroxypropylmethylcellulose, Celluloseacetatphthalat, Polyvinylacetatphthalat, Carboxymethylethylcellulose und Schellack.

8. Verfahren zur Herstellung einer oralen pharmazeutischen Zusammensetzung, wobei das Verfahren umfasst:

   ein erster Schritt der Bildung eines ionischen Komplexes durch Zugabe einer wässrigen Lösung von Desoxycholsäure und Nα-Desoxycholyl-L-lysylmethylester zu einer Lösung, die Teriparatid und Di-alpha-Tocopherolpolyethylenglykol-1000-succinat enthält;
   ein zweiter Schritt der Herstellung einer Mischung durch Zugabe von Caprylocaproyl-Mqacrogol-8-glyceriden als primäres Tensid und Tween 80 als primäres Hilfstensid zur Ionenbindungskomplexlösung;
   einen dritten Schritt zur Herstellung einer Wasser-in-Öl (w/o) primären Nanoemulsion durch Dispergieren der Mischung aus dem zweiten Schritt auf einer primären Ölphase; und
   ein vierter Schritt zur Herstellung einer sekundären Wasser-in-Öl-in-Wasser-Nanoemulsion (w/o/w) durch Zugabe einer Mischung aus Cremopore oder Twin 80 als sekundärem Tensid und Polyethylenglykol 400 als sekundärem Hilfstensid zur primären Wasser-in-Öl-Nanoemulsion (w/o) des dritten Schritts.

**9.** Verfahren nach Anspruch 8, wobei die primäre Ölphase mindestens eine ist, ausgewählt aus der Gruppe bestehend aus Silikonöl, esterbasiertem Öl, kohlenwasserstoffbasiertem Öl, Propylenglykolmonocaprylat, Propylenglykoldicaprylocaprat, Oleoyl-macrogol-6-glyceriden, Lauroylmacrogol-6-Glyceriden, Linolöl-macrogol-6-glyceriden, mittelkettigen Triglyceriden, Ölsäure, Stearinsäure, Glycerylbehenat, Glycerinmonostearat und Rizinusöl.

**10.** Verfahren nach Anspruch 8, wobei die primäre Ölphase in der w/o/w-sekundären Nanoemulsion in einer Menge von 0,1 bis 40 Gew.-%, bezogen auf das Gesamtgewicht der Zusammensetzung, enthalten ist.

**11.** Verfahren nach Anspruch 8, wobei die Mischung aus dem primären Tensid und dem primären Hilfstensid und die Mischung aus dem sekundären Tensid und dem sekundären Hilfstensid in einer Menge von 0,1 bis 40 Gew.-%, bezogen auf das Gesamtgewicht der Zusammensetzung, enthalten sind.

**12.** Verfahren nach Anspruch 8, wobei die primären und sekundären Hilfstenside jeweils unabhängig voneinander mit den primären und sekundären Tensiden in einem Gewichtsverhältnis von 1:0,1 bis 1:10 gemischt werden.

**13.** Verfahren nach Anspruch 8, wobei das Di-alpha-Tocopherol-Polyethylenglykol-1000-Succinat in einer Menge von 0,1 bis 100 Teilen pro 1 Gewichtsteil Teriparatid enthalten ist.

## Revendications

**1.** Composition pharmaceutique orale comprenant un complexe de liaison ionique composé de tériparatide, d'acide désoxycholique, de Nα-désoxycholyl-L-lysyl-méthylester et de succinate de polyéthylène glycol 1000 de di-alpha-tocophérol.

**2.** Composition selon la revendication 1, dans laquelle l'ester de Nα-désoxycholyl-L-lysyl-méthyle est contenu dans 0,1 à 10 moles pour 1 mole de tériparatide.

**3.** Composition selon la revendication 1, dans laquelle l'acide désoxycholique est contenu dans 1 à 20 moles pour 1 mole de tériparatide.

**4.** Composition selon la revendication 1, comprenant en outre un poloxamère.

**5.** Procédé de préparation d'une composition pharmaceutique orale, le procédé comprenant : une première étape consistant à former un complexe ionique par addition d'une solution aqueuse d'acide désoxycholique et de Nα-désoxycholyl-L-lysyl-méthylester à une solution comprenant du tériparatide et du succinate de polyéthylène glycol 1000 de di-alpha-tocophérol ;

une deuxième étape de préparation de granulés par mélange d'un liant, d'un désintégrant, d'un diluant et d'un lubrifiant avec le complexe de liaison ionique préparé dans la première étape ; et
une troisième étape de compression des granulés préparés à la deuxième étape sous forme de comprimés.

**6.** Procédé selon la revendication 5, comprenant en outre une étape consistant à enrober le comprimé préparé dans la troisième étape avec une matière entérique.

**7.** Procédé selon la revendication 6, dans lequel la matière entérique est au moins une matière choisie dans le groupe composé de copolymère acide méthacrylique-acrylate d'éthyle (Eudragit), phtalate d'hydroxypropylméthylcellulose, succinate d'acétyle hydroxypropylméthylcellulose, phtalate d'acétate de cellulose, phtalate d'acétate de polyvinyle, carboxyméthyléthylcellulose. , et de la gomme-laque.

**8.** Procédé de préparation d'une composition pharmaceutique orale, le procédé comprenant :

une première étape consistant à former un complexe ionique par addition d'une solution aqueuse d'acide désoxycholique et de Nα-désoxycholyl-L-lysyl-méthylester à une solution comprenant du tériparatide et du succinate de polyéthylène glycol 1000 de di-alpha-tocophérol ;
une deuxième étape de préparation d'un mélange par addition de glycérides de caprylocaproyl mqacrogol-8 en tant que tensioactif primaire et de Tween 80 en tant que tensioactif auxiliaire primaire à la solution de complexe de liaison ionique ;

une troisième étape de préparation d'une nano-émulsion primaire eau dans huile (e/s) par dispersion du mélange de la deuxième étape sur une phase huileuse primaire ; et

une quatrième étape de préparation d'une nano-émulsion secondaire eau dans huile dans eau (w/o/w) en ajoutant un mélange de Cremopore ou Twin 80 comme tensioactif secondaire et de polyéthylène glycol 400 comme tensioactif auxiliaire secondaire au nano-émulsion primaire eau dans huile (w/o) de la troisième étape.

9. Procédé selon la revendication 8, dans lequel la phase huileuse primaire est au moins une phase choisie dans le groupe composé d'huile de silicone, d'huile à base d'ester, d'huile à base d'hydrocarbure, de monocaprylate de propylène glycol, de dicaprylocaprate de propylène glycol, d'oléoyl macrogol-6 glycérides, de lauroyl macrogol. -6 glycérides, huile linoléique macrogol-6 glycérides, triglycérides à chaîne moyenne, acide oléique, acide stéarique, béhénate de glycéryle, monostéarate de glycérol et huile de ricin.

10. Procédé selon la revendication 8, dans lequel la phase huileuse primaire dans la nano-émulsion secondaire eau/eau est contenue en une quantité de 0,1 à 40 % en poids sur la base du poids total de la composition.

11. Procédé selon la revendication 8, dans lequel le mélange du tensioactif primaire et du tensioactif auxiliaire primaire et le mélange du tensioactif secondaire et du tensioactif auxiliaire secondaire sont contenus en une quantité de 0,1 à 40 % en poids sur la base du poids total de la composition..

12. Procédé selon la revendication 8, dans lequel les tensioactifs auxiliaires primaires et secondaires sont mélangés avec les tensioactifs primaires et secondaires, respectivement, chacun indépendamment dans un rapport pondéral de 1:0,1 à 1:10.

13. Procédé selon la revendication 8, dans lequel le succinate de polyéthylèneglycol 1000 de di-alpha-tocophérol est contenu en une quantité de 0,1 à 100 parties pour 1 partie en poids de tériparatide.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DK 2004000482 W
- WO 2005002549 A1
- CZ 2012000025 W
- WO 2012130193 A1
- CN 102125520 A
- IL 2017050920
- WO 2018033927 A1